# EUROPEAN PATENT APPLICATION

(11) **EP 3 758 017 A1**
(43) Date of publication of application: **30.12.2020**
(21) Application number: 20180917.5
(22) Date of filing: 18.06.2020
(51) Int. Cl.: G16H 40/20

(54) **SYSTEMS AND METHODS FOR COMPLETING ACCEPTED ALERTS**

(30) Priority: 28.06.2019 US 201962868327 P
(71) Applicant: Hill-Rom Services, Inc., Batesville, IN 47006-9167 (US)
(72) Inventor: FRYE, Katherine, Batesville, IN Indiana 47006-9167 (US); KOWAL, Elizabeth A., Batesville, IN Indiana 47006-9167 (US); MOULSON, John D., Batesville, IN Indiana 47006-9167 (US); WOLF, Joseph D., Batesville, IN Indiana 47006-9167 (US); VAN ARK, Jonathan M., Batesville, IN Indiana 47006-9167 (US); MORGAN, Matthew D., Batesville, IN Indiana 47006-9167 (US); NASSAR, Karim, Batesville, IN Indiana 47006-9167 (US)
(74) Representative: Findlay, Alice Rosemary

(57) **Abstract**

A system including an alert owner system and an alert communicator system. The alert owner system may include executable instructions to: generate an alert message by defining at least one acceptable message type attribute acceptable in a type attribute field for alert status update messages. The alert communicator system may include executable instructions to: receive an input after receipt of the alert message, the input including one of a first input or a second input, and wherein: after receiving the first input, present the alert message in an alert list on a GUI, and after receiving the second input: open an audio link to a device at a location associated with the alert message, and generate at least one alert status update message by inserting a message type attribute of the at least one acceptable message type attribute in the type attribute field of each respective alert status update message.

## Description

The present disclosure generally relates to systems and/or methods for completing accepted alerts or caregiver calls (e.g., originating from subjects in a healthcare facility), and more specifically, to systems and/or methods to automate the completing of alerts or caregiver calls accepted via a mobile caregiver application of a caregiver's mobile device as part of a secure caregiver and staff information system.

A mobile caregiver application may be downloaded to a caregiver's mobile device. The caregiver's mobile device may include a wireless communication device provided by a healthcare facility (e.g., usable during the caregiver's shift) and/or a personal cellular phone (e.g., smart phone) of the caregiver. The mobile caregiver application (e.g., a LINQ® Mobile Care Team Application available from Hill-Rom Holdings, Inc. (Batesville, IN)) may function as an alert communicator system and may be used for managing calls and alerts within the healthcare facility as well as for secure voice and text messaging communication between caregivers (e.g., nurses, clinicians, and/or the like), staff (e.g., housekeeping, food services, subject transporting, and/or the like), and subjects. The mobile caregiver application may provide a single mobile communication platform that integrates workflows received from an alert owner system (e.g., a Navicare® Nurse Call system available from Hill-Rom Holdings, Inc. (Batesville, IN)) to improve care team collaboration. Such an alert owner system may provide alerts pertaining to a subject, a room, a bed status, real-time information associated with subject safety and satisfaction, and/or the like.

Although such a mobile caregiver application is able to provide alerts to caregiver mobile devices for acceptance, such a mobile caregiver application is unable to effectively and efficiently cancel a given alert on the alert owner system (e.g., the Navicare® Nurse Call System available from Hill-Rom Holdings, Inc. (Batesville, IN)) and/or clear that given alert on the mobile caregiver application itself (e.g., the LINQ® Mobile Care Team Application available from Hill-Rom Holdings, Inc. (Batesville, IN)). Accordingly, improvements to the mobile caregiver application are desired to effectively and efficiently cancel a particular alert on the alert owner system and/or clear that particular alert from the mobile caregiver application.

In a first aspect, a caregiver information system to generate alert status update messages includes an alert owner system and an alert communicator system. The alert owner system includes: a processor, and a non-transitory memory storing program instructions, the program instructions executable by the processor to: generate an alert message using a predetermined communication protocol, wherein generating the alert message includes defining at least one acceptable message type attribute acceptable in a type attribute field for alert status update messages associated with the alert message. The alert communicator system includes a processor, and a non-transitory memory storing program instructions, the program instructions executable by the processor to: receive, via a graphical user interface, an input after receipt of the alert message, wherein the input includes one of a first input or a second input, and wherein: after receiving the first input, present the alert message in an alert list on the graphical user interface, and after receiving the second input: open an audio link via the alert communicator system to a device at a location associated with the alert message, and generate at least one alert status update message using the communication protocol, wherein generating the at least one alert status update message includes inserting a message type attribute of the at least one acceptable message type attribute in the type attribute field of each respective alert status update message.

In a second aspect, an alert owner system includes: an alert monitoring database, a processor, and a non-transitory memory storing program instructions, the program instructions executable by the processor to: generate an alert message using a predetermined communication protocol, wherein generating the alert message includes defining at least one acceptable message type attribute acceptable in a type attribute field for alert status update messages associated with the alert message, after receiving a closed alert status update message including a first message type attribute: detect the first message type attribute in the type attribute field of the closed alert status update message, and delete the alert message associated with the closed alert status update message from the alert monitoring database, and after receiving an opened-closed alert status update message: detect the second message type attribute in the type attribute field of the opened-closed alert status update message, and delete the alert message associated with the opened-closed alert status update message from the alert monitoring database.

In a third aspect, an alert communicator system, includes: a graphical user interface, a processor, and a non-transitory memory storing program instructions, the program instructions executable by the processor to: receive, via the graphical user interface, an input after receipt of an alert message, wherein the input includes one of a first input or a second input, and wherein: after receiving the first input, present the alert message in an alert list on the graphical user interface, and after receiving the second input: open an audio link to a device at a location associated with the alert message, and generate at least one alert status update message using a predetermined communication protocol, wherein generating the at least one alert status update message includes inserting a message type attribute of at least one acceptable message type attribute in a type attribute field of each respective alert status update message.

In a fourth aspect, a method to generate alert status update messages in a caregiver information system includes: generating, by an alert owner system, an alert message using a predetermined communication protocol, wherein generating the alert message includes defining at least one acceptable message type attribute acceptable in a type attribute field for alert status update messages associated with the alert message. The method further includes: receiving, via a graphical user interface of an alert communicator system, an input after receipt of the alert message, wherein the input includes one of a first input or a second input, and wherein: after receiving the first input, presenting the alert message in an alert list on the on the graphical user interface, and after receiving the second input: opening an audio link via the alert communicator system to a device at a location associated with the alert message, and generating at least one alert status update message using the communication protocol, wherein generating the at least one alert status update message includes inserting a message type attribute of the at least one acceptable message type attribute in the type attribute field of each respective alert status update message.

In a fifth aspect, a method to delete generated alert messages, includes: generating, by an alert owner system, an alert message using a predetermined communication protocol, wherein generating the alert message includes defining at least one acceptable message type attribute acceptable in a type attribute field for alert status update messages associated with the alert message, after receiving a closed alert status update message including a first message type attribute: detecting, by the alert owner system, the first message type attribute in the type attribute field of the closed alert status update message, and deleting, by the alert owner system, the alert message associated with the closed alert status update message from an alert monitoring database, and after receiving an opened-closed alert status update message: detecting, by the alert owner system, the second message type attribute in the type attribute field of the opened-closed alert status update message, and deleting, by the alert owner system, the alert message associated with the opened-closed alert status update message from the alert monitoring database.

The invention will now be further described by way of example with reference to the accompanying drawings, in which:
FIG. 1 depicts a block diagram of an illustrative caregiver and staff information system that depicts a plurality of caregiver mobile devices that execute a mobile caregiver application for managing caregiver calls and alerts originating from subjects and various healthcare facility equipment, according to one or more embodiments of the present disclosure; and
FIG. 2 depicts an illustrative caregiver mobile device including a mobile caregiver application according to one or more embodiments of the present disclosure;
FIG. 3 depicts an illustrative graphical user interface (GUI) screen shot that includes a list of accepted alerts and escalated alerts of a caregiver, according to one or more embodiments shown and described herein;
FIG. 4 depicts an illustrative GUI screen shot that includes a list of accepted alerts and an open alert of a caregiver, according to one or more embodiments shown and described herein;
FIG. 5 depicts an illustrative GUI screen shot that includes options window for a caregiver to "Accept" an alert, to "Accept & Call" the alert, or to "Escalate" the alert, according to one or more embodiments shown and described herein;
FIG. 6 depicts an illustrative GUI screen shot that includes an options window for the caregiver to "Call" an accepted alert or to "Escalate" the accepted alert, according to one or more embodiments shown and described herein;
FIG. 7 depicts a block diagram of illustrative communications between an alert owner system and an alert communicator system, according to one or more embodiments shown and described herein; and
FIG. 8 depicts the illustrative GUI screen shot of FIG. 4 after being modified to include a list of completed alerts of a caregiver, according to one or more embodiments shown and described herein.

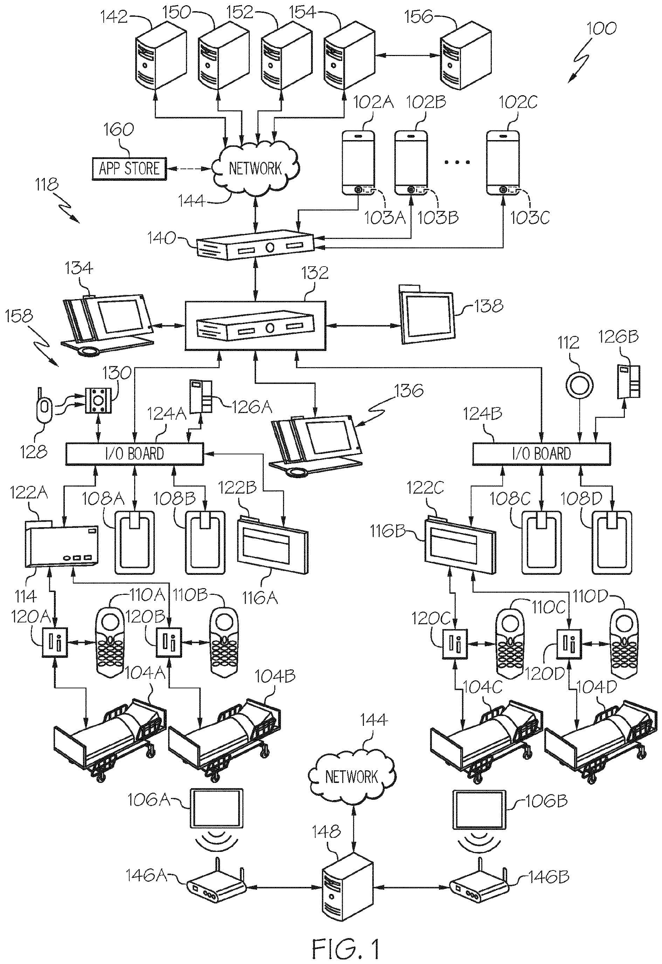

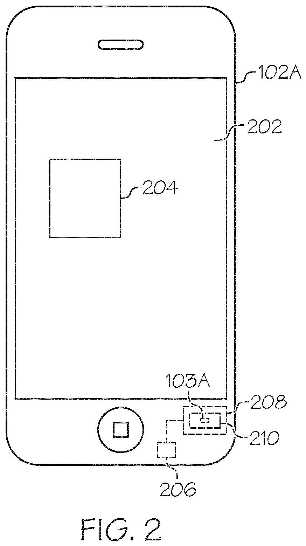

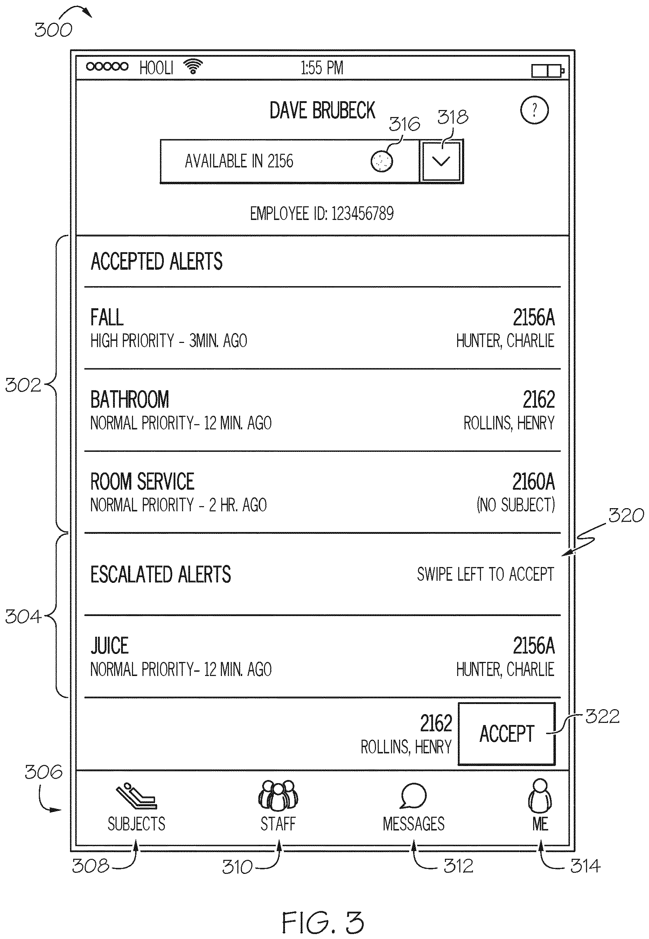

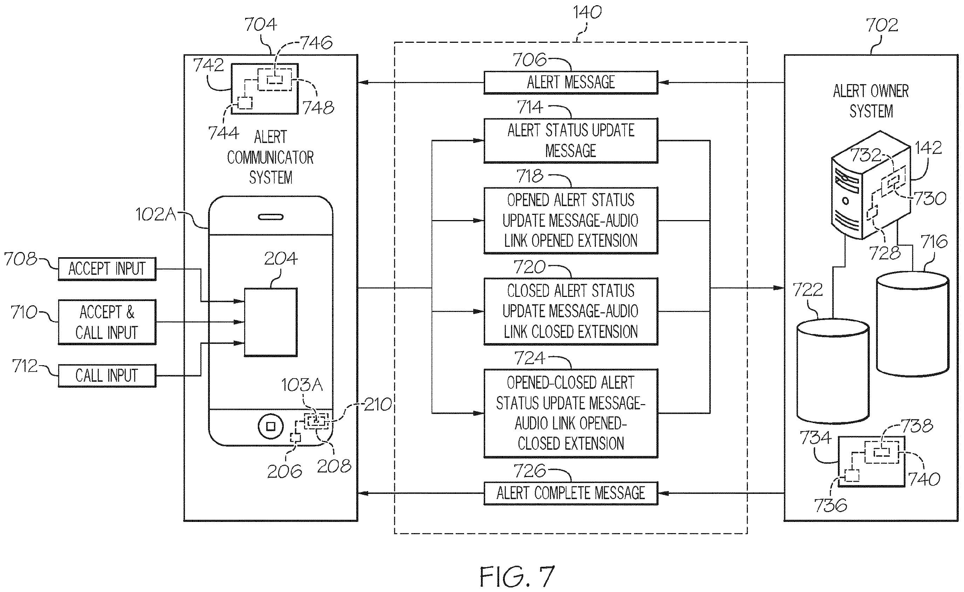

Reference will now be made in detail to systems and/or methods that automate the completing of an alert message after being accepted via a mobile caregiver application of a caregiver's mobile device, examples of which are illustrated in the accompanying drawings. As described herein, completing an alert message may include cancelling a particular alert on an nurse call server of an alert owner system (e.g., a Navicare® Nurse Call system available from Hill-Rom Holdings, Inc. (Batesville, IN)) and/or clearing that particular alert from a mobile caregiver application (e.g., a LINQ® Mobile Care Team Application available from Hill-Rom Holdings, Inc. (Batesville, IN)) of an alert communicator system. Whenever possible, the same or similar reference numerals will be used throughout the drawings to refer to the same or like parts. An illustrative caregiver and staff information system is depicted in FIG. 1. A caregiver and staff information system may generally include a nurse call system having a nurse call server and an alert communicator system having a caregiver mobile device including a processor-executable mobile caregiver application. An illustrative caregiver mobile device including the processor-executable mobile caregiver application is depicted in FIG. 2. Features and functions of the mobile caregiver application are described in connection with at least FIGS. 3-8 which include illustrative GUI screen shots displayable, via the mobile caregiver application, on a user interface screen of a caregiver's mobile device. Features and functions of the nurse call server are described in connection with at least FIG. 7 which depicts illustrative caregiver and staff information system communications between system components.

Turning now to the figures, FIG. 1 depicts a block diagram of an illustrative caregiver and staff information system 100 that depicts a plurality of caregiver mobile devices 102A, 102B, 102C that execute a mobile caregiver application 103A, 103B, 103C, respectively, for managing caregiver calls and alerts originating from subjects and various healthcare facility equipment (e.g., subject beds 104A, 104B, 104C, 104D, subject tablets 106A, 106B, call switches 108A, 108B, 108C, 108D, handheld pillow speaker units 110A, 110B, 110C, 110D and/or the like) located within a subject's room, according to one or more embodiments shown and described herein. In view of FIG. 1, according to some aspects, an alert may also originate from a room sensor 112 (e.g., smoke detector, CO detector, motion detector, and/or the like).

According to various aspects, each mobile caregiver application 103A, 103B, 103C allows a caregiver in an acute care setting to use their respective mobile device 102A, 102B, 102C for managing alerts and caregiver calls from subjects, for conducting voice, video, and text messaging between caregivers, and for permitting voice communications to audio stations (e.g., standard audio station 114 and/or graphical audio stations 116A, 116B) mounted in subject rooms adjacent to respective subject beds 104A, 104B, 104C, 104D. Each mobile caregiver application may act as a secondary notification system that supplements a nurse call system 118 (e.g., a Navicare Nurse Call system available from Hill-Rom Holdings, Inc. (Batesville, IN)) of the caregiver and staff information system 100.

Still referring to FIG. 1, subject beds 104A, 104B, 104C, 104D and respective pillow speaker units 110A, 110B, 110C, 110D may be coupled to respective audio station bed connectors (ASBC's) 120A, 120B, 120C, 120D. In other aspects, one or more network interface units and/or wireless interface units may provide the connectivity between subject beds 104A, 104B, 104C, 104D and a respective standard audio stations 114 and/or graphical audio stations 116A, 116B in lieu of a respective ASBC 120A, 120B, 120C, 120D.

Each of the standard audio stations 114 and/or the graphical audio stations 116A, 116B (e.g., mounted in subject rooms adjacent to respective subject beds 104A, 104B, 104C, 104D) may include a respective code blue call lever 122A, 122B, 122C which may be pulled by a caregiver in an emergency such as when a subject in a room is having a heart attack. Call switches 108A, 108B, 108C, 108D, room sensor 112, standard audio stations 114, and graphical audio stations 116A, 116B may each be communicatively coupled to a respective input/output (I/O) circuit board 124A, 124B. According to various aspects, the input/output circuit boards 124A, 124B may be referred to herein as I/O board or I/O circuitry. Each I/O board 124A, 124B may include a processor (e.g., a microprocessor, microcontroller, and/or the like) that receives various alerts and caregiver calls (e.g., "alert messages") from subject beds 104A, 104B, 104C, 104D, respective pillow speaker units 110A, 110B, 110C, 110D, the room sensor 112, and/or respective standard audio stations 114 and/or graphical audio stations 116A, 116B in response to their respective code blue lever 122A, 122B, 122C being pulled. According to various aspects, the processor of the I/O circuitry 124A, 124B may determine an alert message priority designation for each incoming alert message. According to various aspects, an alert message may be designated as either a normal alert message or a high priority alert message. It should be understood that more than two alert message priority designations may be utilized.

Each I/O board 124A, 124B and therefore, the processor of each I/O board 124A, 124B, may be located at the respective subject room. Thus, the alert message priority designation may be made at each subject room for the alert messages being communicated to each I/O board 124A, 124B. According to such aspects, a central server is not needed for determining message priority for the alert messages received by each I/O board 124A, 124B. According to various aspects, each I/O board 124A, 124B may forward each alert message, and its respective priority designation, to the remainder of the caregiver and staff information system 100.

Each I/O board 124A, 124B may be coupled to a respective dome light 126A, 126B which may include multiple lights that are illuminated to indicate a room status. The illumination of the various lights of each dome light 126A, 126B is controlled by each respective I/O board 124A, 124B based on alert conditions occurring in the respective subject room and based on caregiver presence in, or absence from, the respective subject room (e.g., as determined via the nurse call system 118 as described herein). Dome lights 126A, 126B may be mounted outside each of the subject rooms (e.g., near a doorway to the respective room). In some aspects, each I/O board 124A, 124B may be situated in a housing to which the each respective dome light 126A, 126B may be mounted. In such aspects, each I/O board 124A, 124B may be located outside the respective subject rooms adjacent the respective dome lights 126A, 126B. In other aspects, each I/O board 124A, 124B may be located inside the respective subject rooms. In either case, according to the present disclosure, the I/O boards 124A, 124B are considered to be at the respective subject rooms.

Referring still to FIG. 1, a locating badge 128 may be in wireless communication with a remote locator receiver (RLR) 130 which, in turn, may be communicatively coupled with the respective subject room in which the RLR 130 is located. It should be appreciated that the caregiver and staff information system 100 may include a plurality of locating badges 128 wearable by respective caregivers and a plurality of RLRs 130 located throughout the respective healthcare facility, including being located in the various subject rooms. In response to an RLR 130 detecting one or more locating badge 128 in any particular room, a signal or message may be communicated to a respective I/O board 124A, 124B and the lighting of its respective dome light 126A, 126B is updated accordingly. In the embodiment depicted in FIG. 1, location badge 128 may transmit infrared (IR) signals to RLR 130. In alternative aspects, radio frequency (RF) transmissions, including ultra-wideband (UWB) transmissions, may be made by location badges 128 and/or RLRs 130.

Referring still to FIG. 1, each I/O board 124A, 124B may be communicatively coupled to a Power over Ethernet (PoE) switch 132 which, in turn, may be communicatively coupled to a primary staff console 134 (sometimes referred to as a "master caregiver station"), a secondary staff console 136, and/or a staff terminal 138. PoE switch 132 may be communicatively coupled to a Voice over Internet Protocol (VoIP) Switch and Enterprise server 140 which, in turn, may be communicatively coupled to the nurse call server 142 via a network infrastructure 144. The network infrastructure 144 may include a wide area network (WAN), such as the Internet, a local area network (LAN) such as an Ethernet, a mobile communications network, a public service telephone network (PSTN), a personal area network (PAN), a metropolitan area network (MAN), a virtual private network (VPN), and/or another network. The network infrastructure 144 may electronically connect one or more devices such as computing devices and/or components thereof

According to aspects described herein, various described devices (e.g., call switches 108A, 108B, 108C, 108D, handheld pillow speaker units 110A, 110B, 110C, 110D, standard audio station 114, graphical audio stations 116A, 116B, audio station bed connectors 120A, 120B, 120C, 120D, I/O board 124A, 124B, dome lights 126A, 126B, locating badges 128 remote locator receivers 130, PoE switch 132, primary staff console 134, secondary staff console 136, staff terminal 138, VoIP Switch and Enterprise server 140, nurse call server 142, and/or the like) are illustrative of a diagrammatic nurse call system 118 of the caregiver and staff information system 100. It should be appreciated that the nurse call system 118 architecture may be customized for a particular facility, and thus may vary from one healthcare facility to the next.

According to various aspects, subject tablets 106A, 106B may be included as part of the nurse call system 118 portion of the caregiver and staff information system 100. Subject tablets 106A, 106B may be used by subjects to send specific subject requests (e.g., requests for pain medication, bathroom assistance, food, drink, ice chips, assistance with personal care, and/or the like). According to such aspects, subject tablets 106A, 106B may communicate wirelessly with wireless access points (WAPs) 146A, 146B which, in turn, may communicate with a subject tablet communications server 148. Subject tablet communications server 148 may communicate subject requests, which are also considered to be "alert messages" according to the present disclosure, to the nurse call server 142 via the network infrastructure 144. Accordingly, various described devices (e.g., wireless access points 146A, 146B, subject tablet communications server 148, and/or the like) may further be included as part of the nurse call system 118 portion of the caregiver and staff information system 100.

According to various aspects, alert messages originating from subject beds 104A, 104B, 104C, 104D, may include alert messages relating to one or more of the following: bed exit of the subject from the respective subject bed 104A, 104B, 104C, 104D, subject position on the respective subject bed 104A, 104B, 104C, 104D exceeding a threshold, subject movement on the respective subject bed 104A, 104B, 104C, 104D exceeding a threshold or falling below a threshold, siderail position (e.g., siderail down) of the respective subject bed 104A, 104B, 104C, 104D, wheels of the respective subject bed 104A, 104B, 104C, 104D not being braked, angle of a head section of the respective subject bed 104A, 104B, 104C, 104D being below or above a threshold angle (e.g., 30 degrees), an upper frame of the respective subject bed 104A, 104B, 104C, 104D not being in its lowest position relative to a base frame of the respective subject bed 104A, 104B, 104C, 104D, a bed component exceeding or falling below a threshold temperature, a mattress bladder of the respective subject bed 104A, 104B, 104C, 104D exceeding or falling below a threshold pressure, a pneumatic system error or failure of the respective subject bed 104A, 104B, 104C, 104D, an actuator error or failure of the respective subject bed 104A, 104B, 104C, 104D, an overcurrent condition of a component of the respective subject bed 104A, 104B, 104C, 104D, a power system error or failure of the respective subject bed 104A, 104B, 104C, 104D, and/or power as disconnected from the respective subject bed 104A, 104B, 104C, 104D.

Referring still to FIG. 1, the caregiver and staff information system 100 may further include an electronic medical records (EMR) server 150 and an admission/discharge/transfer (ADT) server 152. The caregiver and staff information system 100 may include various other servers 154 as well. Other servers 154 may include, for example, a real time locating system (RTLS) server 156 that may be communicatively coupled to the remote locator receivers 130 (e.g., via the network infrastructure 144, the VoIP Switch Enterprise Server 140, the PoE switch 132, and the I/O circuit board 124A). In other aspects, the remote locator receivers 130 may not be communicatively coupled to respective I/O boards 124B, 124B. In such aspects, the locating badges 128, the remote locator receivers 130, and the RTLS server 156 may form a real time locating system 158 portion of the caregiver and staff information system 100. In such various aspects, staff location information may be communicated from the RTLS server 156 to the nurse call server 142 via the network infrastructure 144.

In some aspects, the other servers 154 may further include a server that manages the routing of alert messages and related staff information (not shown) to the various caregiver mobile devices 102A, 102B, 102C. In general, alert messages relating to particular subjects or particular rooms assigned to particular caregivers may be sent to the caregiver mobile device 102A, 102B, 102C of that particular caregiver. According to various aspects, the alert messages may originate from subject beds 104A, 104B, 104C, 104D, handheld pillow speaker units 110A, 110B, 110C, 110D, subject tablets 106A, 106B, and/or the like. Alert messages may similarly originate from other types of equipment and may be communicated to the caregiver mobile devices 102A, 102B, 102C within the spirit and scope of the present disclosure.

In some aspects, the mobile caregiver application 103A, 103B, 103C may be available to each caregiver from an Application ("App") Store 160 which may be accessible (e.g., downloadable) via the network infrastructure 144 as indicated by the dashed double headed arrow in FIG. 1. In other aspects, the mobile caregiver application 103A, 103B, 103C may be provided to caregivers internally by a systems administrator of the caregiver and staff information system 100. Thus, in some aspects, the mobile caregiver application 103A, 103B, 103C may be stored in one or more of the servers (e.g., nurse call server 142, subject tablet communications server 148, electronic medical records server 150, admission/discharge/transfer (ADT) server, other servers 154, and/or the like) and is uploadable to the caregiver mobile devices 102A, 102B, 102C and/or downloadable by the caregivers to their respective mobile devices 102A, 102B, 102C. According to some aspects, other caregiver devices and/or interfaces (e.g., primary staff console 134, secondary staff console 136, staff terminal 138, and/or the like) may upload and/or download the mobile caregiver application 103A, 103B, 103C and function similar to a caregiver mobile device 102A, 102B, 102C (e.g., via PoE switch 132), as described herein.

Referring to FIG. 1, various components (e.g., caregiver mobile device 102A, 102B, 102C, primary staff console 134, secondary staff console 136, staff terminal 138, nurse call server 142, EMR server 150, ADT server 152, other servers 154 including RTLS server 156, standard audio stations 114, graphical audio stations 116A, 116B, subject beds 104A, 104B, 104C, 104D, subject tablets 106A, 106B, call switches 108A, 108B, 108C, 108D, handheld pillow speaker units 110A, 110B, 110C, 110D, subject tablet communications server 148, and/or the like) of the caregiver and staff information system 100 may include one or more structural elements including a non-transitory computer-readable medium for completing the various features and/or functionalities described herein, embodied as hardware, software, and/or firmware, according to aspects shown and described herein. According to some aspects, each component may be configured as a general purpose computer with the requisite hardware, software and/or firmware. According to other aspects, each component may be configured as a special purpose computer (e.g., a particular machine) designed specifically to perform the features and/or functionalities as described herein. Here, it should be generally understood that each component may include one computing device or system or a plurality of computing devices or systems. Furthermore, it should be generally understood that each component may include a processor, input/output hardware, network interface hardware, a data storage component and a memory component configured as volatile or non-volatile memory including RAM (e.g., SRAM, DRAM, and/or other types of random access memory), flash memory, registers, compact discs (CDs), digital versatile discs (DVD), and/or other types of storage components. The memory component may include operating logic or program instructions that, when executed, perform the features and/or functionalities described herein. The processor may include any processing component that receives and executes instructions (e.g., operating logic or program instructions from the data storage component and/or memory component) to perform the features and/or functionalities described herein. Network interface hardware may include any wired/wireless hardware generally known to those of skill in the art for communicating with other networks and/or devices.

FIG. 2 depicts an illustrative caregiver mobile device 102A including a respective mobile caregiver application 103A, according to one or more embodiments shown and described herein. Referring to FIG. 2, the caregiver mobile device 102A may include a display screen 202 including a graphical user interface (GUI) 204 disposed thereon. The caregiver mobile device 102A may further include a processor 206 (e.g., as described herein) and a memory 208 (e.g., as described herein) communicatively coupled to the processor 206. The memory 208 stores processor-executable instructions 210 (e.g., as described herein). According to various aspects, the processor-executable instructions 110 may include the mobile caregiver application 103A, as described herein. According to various aspects, the caregiver mobile device 102A, upon execution of the mobile caregiver application 103A from the memory 208 by the processor 206, may be controlled to display various user interfaces and to receive various user inputs/commands via the GUI 204 as described herein.

Illustrative features and functions of the mobile caregiver application 103A, 103B, 103C of the present disclosure, are described below in connection with FIGS. 3-8 which include GUI screen shots shown on the display screen 202 of the mobile device 102A. It should be appreciated that the GUI screen shots of the present disclosure are exemplary in nature and are provided to give a general sense of the type of information that may appear on the display screen 202 of any given mobile device 102A, 102B, 102C during use of the contemplated mobile caregiver application 103A, 103B, 103C. For example, the information such as alert messages, staff names, staff locations, room name formats, unit names, and/or the like is dynamic and may vary from healthcare facility to healthcare facility and, in fact, may vary in any given healthcare facility throughout any given day in response to the various incoming alert messages.

FIG. 3 depicts an illustrative GUI screen shot that includes a list of accepted alerts and escalated alerts of a caregiver, according to one or more embodiments shown and described herein. Referring to FIG. 3, a Staff Detail GUI screen shot 300 includes a list of accepted alerts 302 and escalated alerts 304 of a caregiver. According to various aspects, a main menu 306 may be provided at the bottom of GUI screen 300 and may include a Subjects icon 308, a Staff icon 310, a Messages icon 312, and a Me icon 314. The Staff Detail GUI screen 300 may be presented on the display screen 202 of the respective mobile device 102A in response to the Me icon or button 314 being selected on the main menu 306. As described herein, icons or buttons are considered to be selected or selectable, herein, in that a user touches or taps the icon on the display screen 202 of the respective mobile device 102A to navigate to additional functionality of the mobile caregiver application 103A associated with the respective icon or button.

In the upper section of GUI screen 300 of FIG. 3, the caregiver's name, location and employee identification (ID) number may be presented. In the illustrative example, the employee's name is Dave Brubeck who is located in room 2156 and is available for answering voice calls, phone messages, and alerts. A circle 316 may be provided to the right of the text "Available in 2156" which appears in a text box. The circle 316 may be color coded green if the caregiver is available and may be color coded red if the caregiver is not available. A down arrow icon 318 may be selectable to change the employee's availability (e.g., to "Do Not Disturb" for a time interval, "Sign Out" at the end of a shift, and/or the like).

The accepted alerts 302 of the illustrative GUI screen 300 includes a Fall alert, a Bathroom alert, and a Room Service alert. As depicted in FIG. 3, the Fall alert may be designated as a High Priority alert. Information associated with the Fall alert may indicate when the alert was generated (e.g., 3 min. ago), where the alert was originated (e.g., room 2156A), and who originated the alert (e.g., Charlie Hunter). According to various aspects, the nurse call server 142 may detect a violation of a Falls Protocol based on status information received from a subject bed 104A assigned to Charlie Hunter. In response, the nurse call server 142 may send the Fall alert message (e.g., via the network 144 and the VoIP Switch Enterprise Server 140 of FIG. 1) to the caregiver mobile device 102A assigned to the subject of the subject bed 104A. Similary, the Bathroom alert may be designated as a Normal Priority alert and information associated with the Bathroom alert may indicate when the alert was generated (e.g., 12 min. ago), where the alert was originated (e.g. room 2162), and who originated the alert (e.g., Henry Rollins). According to various aspects, the nurse call server 142 may similarly send the Bathroom alert based on an input received from a subject tablet 106A. Yet further, the Room Service alert may be designated as a Normal Priority alert and information associated with the Room Service alert may indicate when the alert was generated (e.g., 2 hr. ago) and where the alert was originated (e.g. room 2160A). Since no subject is currently assigned to room 2160A, "No Subject" may be displayed. According to various aspects, another caregiver may generate the Room Service alert via their mobile device 102B, 102C or a graphical audio station 116A in room 2160A. According to other aspects, the ADT server 152 may generate the Room Service alert in response to a subject (e.g., previously assigned to room 2160A) being discharged. It should be appreciated that the various alerts may be generated by other caregivers and/or staff members and/or other devices within the caregiver and staff information system 100.

The escalated alerts 304 of the illustrative GUI screen 300 includes a Juice alert. As depicted in FIG. 3, the Juice alert may be designated as a Normal Priority alert and information associated with the Juice alert may indicate when the alert was generated (e.g., 12 min. ago), where the alert originated (e.g., room 2156A), and who originated the alert (e.g., Charlie Hunter). In view of FIG. 3, the primary caregiver, who is a caregiver other than Dave Brubeck, was not able to respond to this particular alert message and so the alert was escalated from that other caregiver to Dave Brubeck (e.g., a secondary caregiver) for possible response. The secondary caregiver is the caregiver designated for escalation of any alerts to which a primary caregiver is unable to respond. It is contemplated by this disclosure that escalation of alerts may occur in various manners such as, for example, after a preset period of time has elapsed without acceptance by the primary caregiver and/or if the primary caregiver has a status of unavailable and/or if the primary caregiver uses their mobile device 102A, 102B, 102C to manually escalate an alert to a secondary caregiver. Still referring to FIG. 3, a "swipe left to accept" icon 320 may be provided on the GUI screen 300 and may be used by the caregiver to accept any incoming escalated alerts by swiping left on the icon 320 or by swiping left on the escalated alert (e.g., Juice alert) information itself. In the illustrative example of FIG. 3, yet another alert is originating from room 2162 of subject Henry Rollins and can be accepted by the caregiver by selecting the Accept button 322.

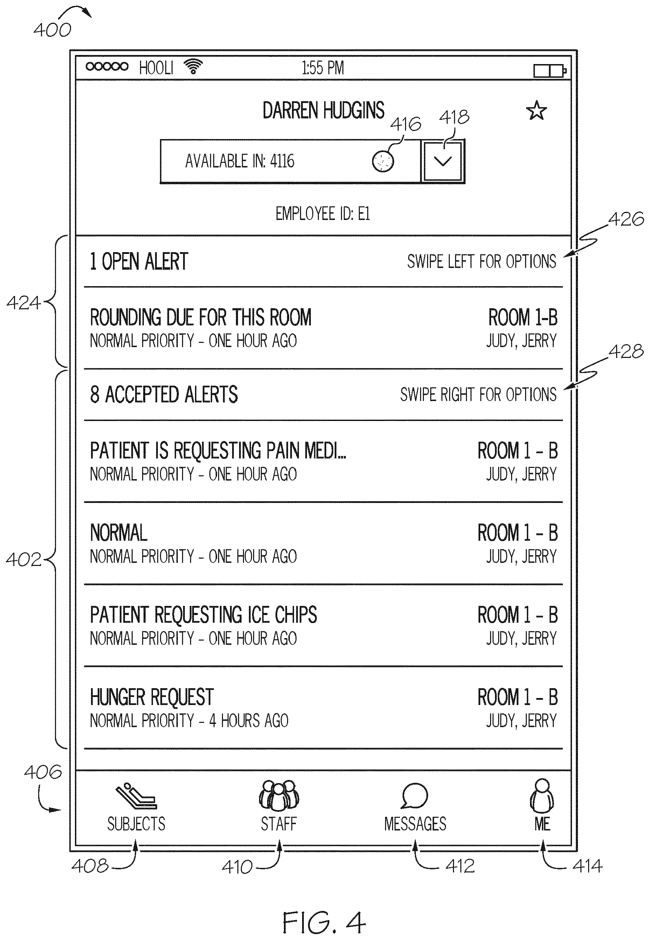

FIG. 4 depicts another illustrative Staff Detail GUI screen shot 400 that includes a list of accepted alerts 402 and an open alert 424 of a caregiver, according to one or more embodiments shown and described herein. Similar to FIG. 3, the GUI screen 400 may be presented on the display screen 202 of the respective mobile device 102A in response to the Me icon or button 414 being selected on the main menu 406. The GUI screen 400 may also include a Subjects icon 408, a Staff icon 410, and a Messages icon 412. The upper section of the GUI screen 400 similarly includes the caregiver's name, location and employee identification (ID) number. In the illustrative example of FIG. 4, the employee's name is Darren Hudgins, who is located in room 4116 and is available for answering voice calls, phone messages, and alerts as indicated by circle 416 being color coded green. The GUI screen 400 may also include a down arrow icon 418 to change to employee's availability.

Continuing the illustrative example, the open alert 424 of the illustrative GUI screen 400 includes a Rounding alert (e.g., a caregiver's need to visit a subject(s) assigned to them as part of their rounds in the facility). As depicted in FIG. 4, the Rounding alert may be designated as a Normal Priority alert and information associated with the Rounding alert may indicate when the alert was generated (e.g., one hour ago), what room is associated with the alert (e.g., Room 1-B), and who is assigned to the room associated with the alert (e.g., Jerry Judy). According to various aspects, the Rounding alert may be originated from one or more of the nurse call server 142, the EMR server 150, and other server(s) 154 (e.g., FIG. 1). In particular, a "swipe left for options" icon 426 may be presented to the right of the text "1 OPEN ALERT" and may be used by the caregiver to pull up an options window by swiping left on the icon 426 or by swiping left on the open alert (e.g., Rounding alert) information itself. The options window associated with icon 426 may provide the caregiver with buttons for accepting the open alert or escalating the open alert to another caregiver. In some aspects, the options window associated with icon 426 may also include a button for accepting the alert and calling the subject's room. Similarly, a "swipe right for options" icon 428 may be presented to the right of the text "8 ACCEPTED ALERTS" and may be used by the caregiver to pull up an options window by swiping right on the icon 428 or by swiping right on any accepted alert (e.g., Hunger Request alert) information itself. The options window associated with icon 428 may provide the caregiver a button for escalating the accepted alert to another caregiver or for calling the subject's room.

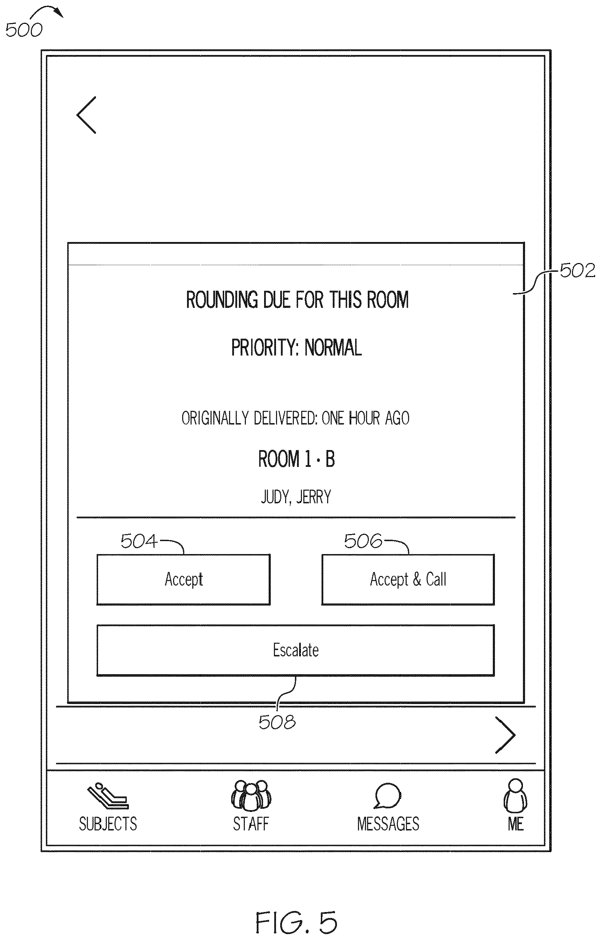

FIG. 5 depicts an illustrative GUI screen shot 500 that includes an options window 502 for the caregiver to "Accept" an alert, to "Accept & Call" the alert, or to "Escalate" the alert, according to one or more embodiments shown and described herein. The options window 502 may be presented on the display screen 202 of the respective mobile device 102A, for example, in response to a caregiver swiping left on icon 426 or swiping left on an open alert itself as described in FIG. 4. In view of FIG. 5, the options window 502 may include an upper section including further information about an incoming alert. In the illustrative example, the subject's name (e.g., Jerry Judy) is included in the options window 502 along with the alert type (e.g., Rounding alert), alert priority (e.g., Normal), and delivery time (e.g., one hour ago). A lower section of the options window 502 may include an Accept icon 504, an Accept & Call icon 506, and an Escalate icon 508. In response to selection of the Accept icon 504, the caregiver accepts responsibility for responding to the alert and the alert message in the upper section of options window 502 may be added to the list of the caregiver's accepted alerts (e.g., FIG. 4, reference 402). Still referring to FIG. 5, in response to selection of the Accept and Call icon 506, the caregiver not only accepts responsibility for responding to the alert, which may be added to the list of the caregiver's accepted alerts (e.g., FIG. 4, reference 402) but also a voice call is placed to the room (e.g., Room 1-B) of the subject from which the alert originated. Referring to FIGS. 1 and 5, according to various aspects, the voice call may be placed via a standard audio station 114 or a graphical audio stations 116A, 116B to speak with the subject (e.g., via a pillow speaker 110A, 110B, 110C, 110D or the like). For example, referring to FIG. 1, a voice call may be established between a caregiver mobile device 102C and a pillow speaker 110C associated with a subject in subject bed 104C (e.g., from caregiver mobile device 102C, through VoIP Switch and Enterprise server 140, PoE switch 132, I/O board 124B, graphical audio station 116B, and ASBC 120C to pillow speaker 110C). Lastly, in response to selection of the Escalate icon 508, the alert may be escalated to another caregiver such as an assigned secondary caregiver for the subject.

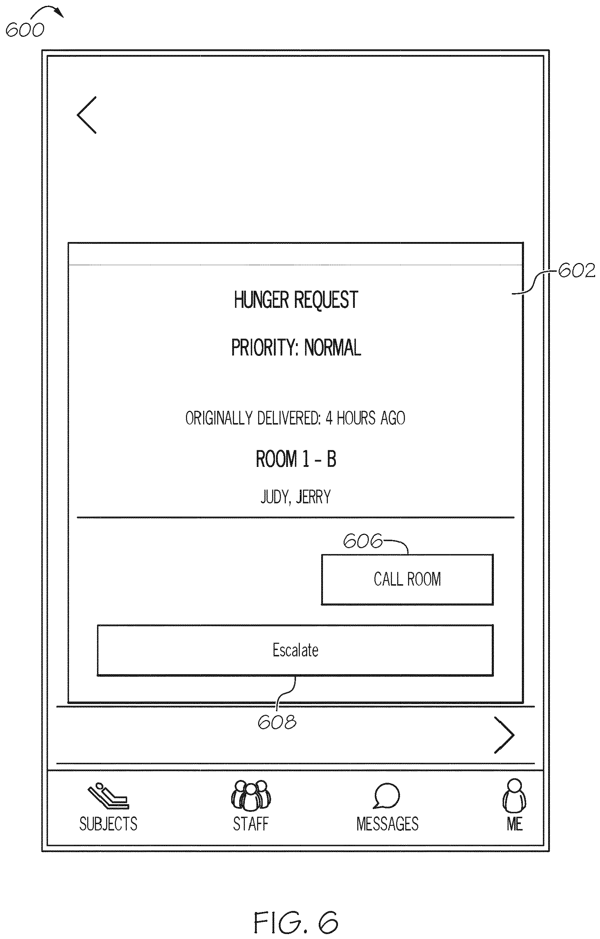

FIG. 6 depicts an illustrative GUI screen shot 600 that includes an options window 602 for the caregiver to "Call" an accepted alert or to "Escalate" the accepted alert, according to one or more embodiments shown and described herein. The options window 602 may be presented on the display screen 202 of the respective mobile device 102A, for example, in response to a caregiver swiping right on icon 428 or swiping right on any accepted alert itself as described in FIG. 4. In view of FIG. 6, the options window 602 may include an upper section including further information about an incoming alert. In the illustrative example, the subject's name (e.g., Jerry Judy) is included in the options window 602 along with the alert type (e.g., Hunger Request alert), alert priority (e.g., Normal), and delivery time (e.g., 4 hours ago). A lower section of the options window 602 may include a Call Room icon 606, and an Escalate icon 608. In response to selection of the Call Room icon 606, a voice call may be placed to the room (e.g., Room 1-B) of the subject from which the alert originated. Referring to FIGS. 1 and 6, according to various aspects, the voice call may be placed via a standard audio station 114, a graphical audio stations 116A, 116B to speak with the subject (e.g., via a pillow speaker 110A, 110B, 110C, 110D or the like). In response to selection of the Escalate icon 608, the alert may be escalated to another caregiver, such as an assigned secondary caregiver for the subject.

Within this backdrop of FIGS. 1-6, systems and/or methods that automate the completing of an accepted alert message are described. Here, completing an alert message may include cancelling a particular alert on a nurse call server 142 of an alert owner system and/or clearing that particular alert from a mobile caregiver application 103A of an alert communicator system. Such an automated completing of accepted alert messages creates a seamless workflow between the alert owner system and the alert communicator system.

According to various aspects of the present disclosure, after an audio connection is opened to a location of a particular alert's origination (e.g., after selection of an associated Accept and Call icon 506 of FIG. 5 and/or an associated Call Room icon 606 of FIG. 6) the systems and/or methods of the present disclosure may cancel that particular alert on a nurse call server 142 of an alert owner system and/or clear that particular alert on a mobile caregiver application 103A of an alert communicator system.

FIG. 7 depicts a block diagram of illustrative communications between an alert owner system 702 and an alert communicator system 704, according to one or more embodiments shown and described herein. According to aspects of the present disclosure, such communications may utilize Wireless Communications Transfer Protocol (WCTP). WCTP is a transfer protocol that uses transport protocols including Hypertext Transfer Protocol (HTTP) and standards including Extensible Markup Language (XML) to transfer information between wire line and wireless systems. Details regarding the basic elements, components, and functionality of WCTP (e.g., Header, Payload, Confirmation Response, Response Header, Notification, and/or the like) are generally understood and are beyond the scope of the present disclosure. According to various aspects, the illustrative communications may occur at the alert owner system 702 via a processor executing program instructions stored in a non-transitory memory (e.g., via a processor 728 of the nurse call server 142 executing program instructions 730 stored in memory 732, via a processor 736 of another component 734 (see FIG. 1) of the alert owner system 702 executing program instructions 738 stored in memory 740, and/or the like) and at the alert communicator system 704 via a processor executing program instructions stored in a non-transitory memory (e.g., via a processor 206 of the caregiver mobile device 102A executing mobile caregiver application 103A stored in memory 208, via a processor 744 of another component 742 (see FIG. 1) of the alert communicator system 704 executing program instructions 746 stored in memory 748, and/or the like). For purposes of simplification, FIG. 7 illustrates the various alert messages described herein as transmitted directly between the alert owner system 702 and the alert communicator system 704. However, in view of FIG. 1, the various alert messages may be indirectly communicated from the nurse call server 142 of the alert owner system 702 to the caregiver mobile device 102A of the alert communicator system 704, and vice versa, for example, via the VoIP Switch and Enterprise server 140 (FIG. 7, shown in phantom, e.g., a WCTP server) and its delivering network (FIG. 1, e.g., network infrastructure 144). According to some aspects, the VoIP Switch and Enterprise server 140 may include an alert management service having a WCTP provider/server (e.g., HMS).

According to aspects of the present disclosure, a Notification element of a WCPT message (e.g., sent by a message originator) may specify acceptable or valid values (e.g., enumeration(s) of characters with a predetermined maximum and minimum length) for its type attribute (e.g., wctp-Notificaton.type field) for a notification status response (e.g., an alert status update message). Standard type attributes include "QUEUED", "DELIVERED" and "READ". However, with respect to the Notification element, aspects of the present disclosure define or expand the acceptable or valid values for the type attribute field. More specifically, expanded values for the type attribute field, as described herein, may include a value to indicate that an audio link has been opened (e.g., "IHEPCDCALLBACKSTART", "IHEPCDCALLBACKOPEN"), a value to indicate that the audio link has been closed (e.g., "IHEPCDCALLBACKEND"), and a value to indicate that the audio link has been opened-closed (e.g., "IHEPCDCALLSTARTEND"), and/or the like. Here, it should be appreciated that such specific enumerated character strings are illustrative and non-limiting to the present disclosure. As such, according to aspects described herein, the VoIP Switch and Enterprise server 140 and its network infrastructure 144 (e.g., FIG. 1), a WCTP provider/server of an alert management service and/or the like, recognizes such expanded values for the type attribute field and generates and relays response messages (e.g., in accordance with WCPT protocol, setting a correct value in the wctp-Notification.type field, and/or the like) between the nurse call server 142 of the alert owner system 702 to the caregiver mobile device 102A of the alert communicator system 704.

Referring to FIG. 7, the alert owner system 702 transmits an alert message 706 to the alert communicator system 704. Here, according to aspects of the present disclosure, the alert message 706 may be transmitted using WCTP. More specifically, as described herein, the alert message 706 may specify acceptable or valid values for its type attribute (e.g., for a notification status response) to include not only standard "QUEUED", "DELIVERED" and "READ" but also expanded "IHEPCDCALLBACKS TART", "IHEPCDCALLBACKEND", "IHEPCDCALLSTARTEND", and/or the like, type attributes. In view of FIG. 7, the alert communicator system 704 may direct the alert message 706 to a caregiver mobile device 102A.

As described herein, various inputs may be received via the caregiver mobile device 102A (e.g., via GUI 204) in response to the alert message 706. More specifically, an Accept input 708 (e.g., via Accept icon 504 of FIG. 5) may be received, an Accept & Call input 710 (e.g., via Accept & Call icon 506 of FIG. 5) may be received, or a Call input 712 (e.g., via Call Room icon 606 of FIG. 6) may be received via a mobile caregiver application 103A of the caregiver mobile device 102A.

### No Alert Owner System Cancellation

According to one aspect of the present disclosure, if an Accept input 708 is received, the mobile caregiver application 103A adds the alert message 706 to the caregiver's list of accepted alerts (e.g., FIG. 4, reference 402). Further in such an aspect, if an Accept & Call input 710, or a Call input 712 is received, the mobile caregiver application 103A opens a two-way audio link on the caregiver mobile device 102A to a location (e.g., device in subject room) associated with the alert message 706 (e.g., VoIP connection). In response to and/or after receipt of the Accept input 708, the Accept & Call input 710, or the Call input 712, the mobile caregiver application 103A and/or the alert communicator system 704 transmits an alert status update message 714 to the alert owner system 702. The alert status update message 714 may be transmitted using WCTP. More specifically, the mobile caregiver application 103A and/or the alert communicator system 704 transmits the alert status update message 714 including the standard "QUEUED", "DELIVERED" and/or "READ" type attributes to confirm, to the alert owner system 702, that the alert message 706 has been successfully communicated (e.g., queued, delivered, read, and/or the like). In response to and/or after the alert status update message 714 is received, a nurse call server 142 of the alert owner system 702 saves a record in its alert alert monitoring database 716 (e.g., in association with the alert message 706) to document that the alert message 706 has been successfully communicated. According to such an aspect, however, the alert message 706 is not cancelled (e.g., cleared, deleted) as a record from the alert alert monitoring database 716.

### Alert Owner System Cancellation, Opened Alert Status Update Message and Closed Alert Status Update Message

According to another aspect of the present disclosure, if an Accept & Call input 710, or a Call input 712 is received, the mobile caregiver application 103A opens a two-way audio link on the caregiver mobile device 102A to a location (e.g., device in subject room) associated with the alert message 706 (e.g., VoIP connection). In response to and/or after receipt of the Accept & Call input 710 or the Call input 712, the mobile caregiver application 103A and/or the alert communicator system 704 transmits an opened alert status update message 718 to the alert owner system 702. The opened alert status update message 718 may be transmitted using WCTP. In particular, the mobile caregiver application 103A and/or the alert communicator system 704 modifies the alert status update message 714 to include a first predetermined WCTP extension in a particular field (e.g., wctp-Notificaton.type field) to indicate that an audio communication has been opened to the location associated with the alert message 706. More specifically, the mobile caregiver application 103A and/or the alert communicator system 704 transmits the opened alert status update message 718 including a type attribute to indicate that an audio link has been opened (e.g., "IHEPCDCALLBACKSTART") to confirm, to the alert owner system 702, that an audio communication has been opened to the location (e.g., device in subject room) associated with the alert message 706. In response to and/or after the opened alert status update message 718 is received, a nurse call server 142 of the alert owner system 702 detects the first predetermined WCTP extension (e.g., "IHEPCDCALLBACKSTART") in the particular field (e.g., wctp-Notificaton.type field) and to save a record in its alert monitoring database 716 to document that the audio communication has been opened to the location associated with the alert message 706. Further, in such an aspect, the mobile caregiver application 103A and/or the alert communicator system 704 monitors the open two-way audio link (e.g., VoIP connection). In response to and/or after detecting that the open two-way audio link has been closed (e.g., by the caregiver mobile device 102A, by a device in the subject room as described herein, and/or the like, VoIP disconnected after successful connection), the mobile caregiver application 103A and/or the alert communicator system 704 transmits a closed alert status update message 720 to the alert owner system 702. The closed alert status update message 720 may be transmitted using WCTP. In particular, the mobile caregiver application 103A and/or the alert communicator system 704 modifies the alert status update message 714 to include a second predetermined WCTP extension in a particular field (e.g., wctp-Notificaton.type field) to indicate that the audio communication has been closed to the location associated with the alert message 706. More specifically, the mobile caregiver application 103A and/or the alert communicator system 704 transmits the closed alert status update message 720 including a type attribute to indicate that an audio link has been closed (e.g., "IHEPCDCALLBACKEND") to confirm, to the alert owner system 702, that the audio communication has been closed to the location (e.g., device in subject room) associated with the alert message 706. In response to and/or after the closed alert status update message 720 is received, a nurse call server 142 of the alert owner system 702 detects the second predetermined WCTP extension (e.g., "IHEPCDCALLBACKEND") in the particular field (e.g., wctp-Notificaton.type field) and to match the closed alert status update message 720 to its corresponding opened alert status update message 718 in the alert monitoring database 716 (e.g., associated with the same alert message 706). Further, in such an aspect, the nurse call server 142 of the alert owner system 702 cancels (e.g., clears, deletes) the alert message 706 and all associated status update messages (e.g., matching opened alert status update message 718 and closed alert status update message 720, alert status update message 714 if an Accept input 708 is initially received as described below, and/or the like) from the alert monitoring database 716 and to generate a record in its alert completed database 722 to document that the alert message 706 has been satisfied. Further in such an aspect, if an Accept input 708 is initially received, the mobile caregiver application 103A adds the alert message 706 to the caregiver's list of accepted alerts (e.g., FIG. 4, reference 402). In response to and/or after receipt of the Accept input 708, the mobile caregiver application 103A and/or the alert communicator system 704 transmits an alert status update message 714 to the alert owner system 702 and the alert owner system 702 saves a record in its alert monitoring database 716 (e.g., in association with the alert message 706) to document that the alert message 706 has been successfully communicated. Here, in response to and/or after a Call input 712 (e.g., FIG. 6, reference 606) is received, the mobile caregiver application 103A and/or the alert communicator system 704 transmits the opened alert status update message 718 and/or closed alert status update message 720 and the nurse call server 142 of the alert owner system cancels (e.g., clears, deletes) the alert message 706 and all associated status update messages (e.g., matching opened alert status update message 718, closed alert status update message 720, and alert status update message 714) and to generate a record in its alert completed database 722.

### Alert Owner System Cancellation, Opened-Closed (Opened and Closed) Alert Status Update Message

According to yet another aspect of the present disclosure, if an Accept & Call input 710, or a Call input 712 is received, the mobile caregiver application 103A opens a two-way audio link on the caregiver mobile device 102A to a location (e.g., device in subject room) associated with the alert message 706 (e.g., VoIP connection). In response to and/or after receipt of the Accept & Call input 710 or the Call input 712, the mobile caregiver application 103A and/or the alert communicator system 704 may hold the opened alert status update message 718 (e.g., not transmit the opened alert status update message 718 to the alert owner system 702). In such an aspect, the mobile caregiver application 103A and/or the alert communicator system 704 monitors the open two-way audio link (e.g., VoIP connection). In response to and/or after detecting that the open two-way audio link has been closed (e.g., by the caregiver mobile device 102A, by a device in the subject room as described herein, and/or the like, VoIP disconnected after successful connection), the mobile caregiver application 103A and/or the alert communicator system 704 transmits an opened-closed alert status update message 724 to the alert owner system 702. The opened-closed alert status update message 724 may be transmitted using WCTP. In particular, the mobile caregiver application 103A and/or the alert communicator system 704 modifies the alert status update message 714 to include a third predetermined WCTP extension in a particular field (e.g., wctp-Notificaton.type field) to indicate that the audio communication has been opened and closed to the location associated with the alert message 706. More specifically, the mobile caregiver application 103A and/or the alert communicator system 704 transmits the opened-closed alert status update message 724 including a type attribute to indicate that an audio link has been opened and closed (e.g., "IHEPCDCALLSTARTEND") to confirm, to the alert owner system 702, that the audio communication has been opened and closed to the location (e.g., device in subject room) associated with the alert message 706. In response to and/or after the opened-closed alert status update message 724 is received, a nurse call server 142 of the alert owner system 702 detects the third predetermined WCTP extension (e.g., "IHEPCDCALLSTARTEND") in the particular field (e.g., wctp-Notificaton.type field) and to match the opened-closed alert status update message 724 to its associated alert message 706 in the alert monitoring database 716. Further, in such an aspect, the nurse call server 142 of the alert owner system 702 cancels (e.g., clears, deletes) the alert message 706 and all associated status update messages (e.g., opened-closed alert status update message 724, alert status update message 714 if an Accept input 708 is initially received as described below, and/or the like) from the alert monitoring database 716 and to generate a record in its alert completed database 722 to document that the alert message 706 has been satisfied. Further in such an aspect, if an Accept input 708 is initially received, the mobile caregiver application 103A adds the alert message 706 to the caregiver's list of accepted alerts (e.g., FIG. 4, reference 402). Similar to above, in response to and/or after receipt of the Accept input 708, the mobile caregiver application 103A and/or the alert communicator system 704 transmits an alert status update message 714 to the alert owner system 702 and the alert owner system 702 saves a record in its alert monitoring database 716 (e.g., in association with the alert message 706) to document that the alert message 706 has been successfully communicated. Here, in response to and/or after a Call input 712 (e.g., FIG. 6, reference 606) is received, the mobile caregiver application 103A and/or the alert communicator system 704 transmits the opened-closed alert status update message 724 and the nurse call server 142 of the alert owner system cancels (e.g., clears, deletes) the alert message 706 and all associated status update messages (e.g., opened-closed alert status update message 724 and alert status update message 714) and to generate a record in its alert completed database 722.

### Alert Owner System Cancellation, Opened Alert Status Update Message

According to a further aspect of the present disclosure, if an Accept & Call input 710, or a Call input 712 is received, the mobile caregiver application 103A opens a two-way audio link on the caregiver mobile device 102A to a location (e.g., device in subject room) associated with the alert message 706 (e.g., VoIP connection). In response to and/or after receipt of the Accept & Call input 710 or the Call input 712, the mobile caregiver application 103A and/or the alert communicator system 704 transmits an opened alert status update message 718 to the alert owner system 702. The opened alert status update message 718 may be transmitted using WCTP. In particular, the mobile caregiver application 103A and/or the alert communicator system 704 modifies the alert status update message 714 to include a fourth predetermined WCTP extension in a particular field (e.g., wctp-Notificaton.type field) to indicate that an audio communication has been opened to the location associated with the alert message 706. More specifically, the mobile caregiver application 103A and/or the alert communicator system 704 transmits the opened alert status update message 718 including a type attribute to indicate that an audio link has been opened (e.g., "IHEPCDCALLBACKOPEN") to confirm to the alert owner system 702 that an audio communication has been opened to the location (e.g., subject room) associated with the alert message 706. In response to and/or after the opened alert status update message 718 is received, a nurse call server 142 of the alert owner system 702 detects the fourth predetermined WCTP extension (e.g., "IHEPCDCALLBACKOPEN") in the particular field (e.g., wctp-Notificaton.type field) and to match the opened alert status update message 718 to its associated alert message 706 in the alert monitoring database 716. Further, in such an aspect, the nurse call server 142 of the alert owner system 702 cancels (e.g., clears, deletes) the alert message 706 and all associated status update messages (e.g., opened alert status update message 718, alert status update message 714 if an Accept input 708 is initially received as described herein, and/or the like) from the alert monitoring database 716 and to generate a record in its alert completed database 722 to document that the alert message 706 has been satisfied. Further, in such an aspect, if an Accept input 708 is initially received, the mobile caregiver application 103A adds the alert message 706 to the caregiver's list of accepted alerts (e.g., FIG. 4, reference 402). Similar to above, in response to and/or after receipt of the Accept input 708, the mobile caregiver application 103A and/or the alert communicator system 704 transmits an alert status update message 714 to the alert owner system 702 and the alert owner system 702 saves a record in its alert monitoring database 716 (e.g., in association with the alert message 706) to document that the alert message 706 has been successfully communicated. Here, in response to and/or after a Call input 712 (e.g., FIG. 6, reference 606) is received, the mobile caregiver application 103A and/or the alert communicator system 704 transmits the opened alert status update message 718 and the nurse call server 142 of the alert owner system cancels (e.g., clears, deletes) the alert message 706 and all associated status update messages (e.g., opened alert status update message 718 and alert status update message 714) and to generate a record in its alert completed database 722, as just described. In such an aspect, the mobile caregiver application 103A and/or the alert communicator system 704 may not monitor the open two-way audio link, detect that the open two-way audio link has been closed, and/or transmit a closed alert status update message 724 and the nurse call server 142 of the alert owner system 702 may not detect a further predetermined WCTP extension and/or match the open alert status update message 718, as described herein.

### Alert Communicator System, Alert Completion

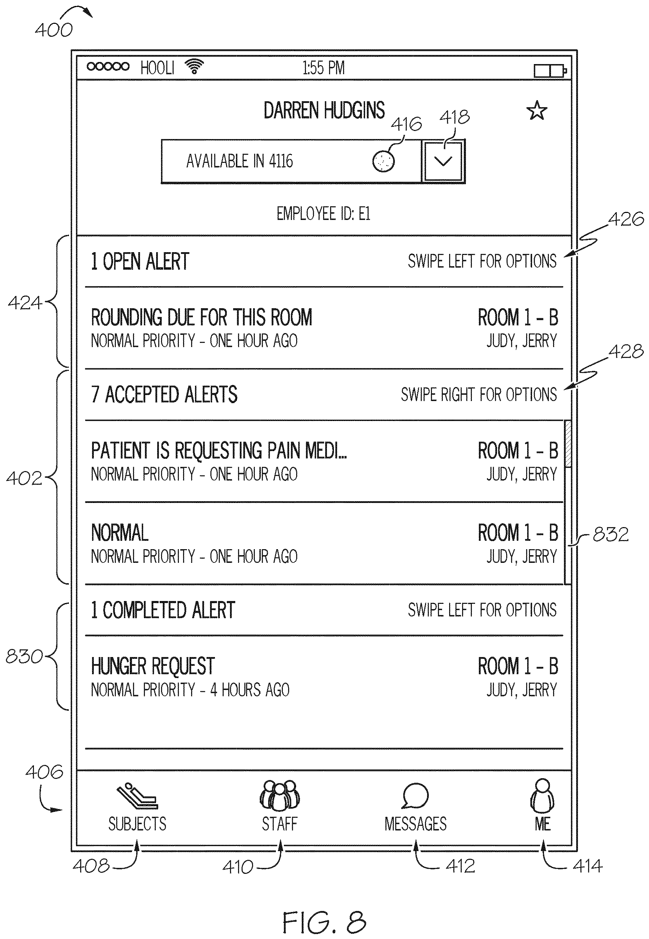

Referring still to FIG. 7, the nurse call server 142 of the alert owner system 702, in response to and/or after cancelling the alert message 706 and all associated status update messages (e.g., as described herein) from the alert monitoring database 716 and/or generating a record in its alert completed database 722 (e.g., as described herein) generates and transmits an alert complete message 726 (e.g., associated with the alert message 706) to the alert communicator system 704. Here, according to aspects of the present disclosure, the alert complete message 726 may be transmitted using WCTP (e.g., similar to alert status update message 714) and the alert communicator system 704 may direct the alert complete message 726 to the caregiver mobile device 102A. In response to and/or after receipt of the alert complete message 726, the mobile care application 103A removes the alert message 706 from the caregiver's list of accepted alerts (e.g., FIG. 4, reference 402). Furthermore, according to various aspects, the mobile caregiver application 103A moves the alert message 706 to a list of completed alerts (e.g. FIG. 8).

FIG. 8 depicts the illustrative Staff Detail GUI screen shot 400 of FIG. 4 after being modified to include a list for completed alerts 830 of a caregiver, according to one or more embodiments shown and described herein. In the illustrative example, referring briefly to FIG. 4 and FIG. 6, a caregiver has swiped right on an accepted alert (e.g., Hunger Request alert) from the caregiver's list of accepted alerts 402 (e.g., FIG. 4) and has selected the Call Room icon 606 (FIG. 6) to open a two-way audio link on the caregiver's mobile device 102A to a location (e.g., Room 1-B) associated with the Hunger Request alert message (e.g., VoIP connection). In response to and/or after receipt of an alert complete message (FIG. 7, reference 726) associated with the Hunger Request alert, the mobile care application 103A removes the Hunger Request alert message from the caregiver's list of accepted alerts (e.g., FIG. 8, reference 402). In particular, the GUI screen 400 may be dynamically modified to reflect a new count of accepted alerts (e.g, "7 ACCEPTED ALERTS"). Further in view of FIG. 8, the GUI screen 400 may be further modified to include a list of completed alerts 830 as well as reflect a new count of completed alerts (e.g., "1 COMPLETED ALERT"). According to various aspects, in order to present a list of completed alerts, one or more sections of the GUI screen 400 may be dynamically modified to include a scroll bar 832 for the caregiver to scroll through alerts (e.g., open alerts, accepted alerts, completed alerts, and/or the like).

It should now be understood that the systems and/or methods described herein are suitable not only for automatically cancelling a particular alert message on an alert owner system (e.g., Navicare® Nurse Call System available from Hill-Rom Holdings, Inc. (Batesville, IN)) but also for automatically completing the particular alert message on an alert communicator system (e.g., LINQ® Mobile Care Team Application available from Hill-Rom Holdings, Inc. (Batesville, IN)). The methods and systems of the present disclosure expand the functional utility of alert messages to enable the effective and efficiently cancellation of the particular alert on a nurse call server of the alert owner system and/or completion of the particular alert on a mobile caregiver application of the alert communicator system. More specifically, the methods and systems of the present disclosure not only define acceptable type attributes (e.g., beyond standard type attributes) for WCTP alert status update messages but also utilize such acceptable type attributes, in particular ways, to automatically delete a particular alert message from being further monitored on a nurse call server and to automatically complete accepted alerts on a GUI of a caregiver's mobile device.

While particular embodiments have been illustrated and described herein, it should be understood that various other changes and modifications may be made. Moreover, although various aspects have been described herein, such aspects need not be utilized in combination.

Embodiments of the invention can be described with reference to the following numbered clauses, with preferred features laid out in the dependent clauses:
1. A caregiver information system to generate alert status update messages, the system comprising:
   an alert owner system, including:
      a processor; and
      a non-transitory memory storing program instructions, the program instructions executable by the processor to:
         generate an alert message using a predetermined communication protocol, wherein generating the alert message includes defining at least one acceptable message type attribute acceptable in a type attribute field for alert status update messages associated with the alert message; and
   an alert communicator system, including:
      a processor; and
      a non-transitory memory storing program instructions, the program instructions executable by the processor to:
         receive, via a graphical user interface, an input after receipt of the alert message, wherein the input includes one of a first input or a second input, and wherein:
         after receiving the first input, present the alert message in an alert list on the graphical user interface; and
         after receiving the second input:
            open an audio link via the alert communicator system to a device at a location associated with the alert message; and
            generate at least one alert status update message using the communication protocol, wherein generating the at least one alert status update message includes inserting a message type attribute of the at least one acceptable message type attribute in the type attribute field of each respective alert status update message.
2. The system of clause 1, wherein the at least one acceptable message type attribute includes a first message type attribute to indicate that the audio link has been closed, a second message type attribute to indicate that the audio link has been opened and closed, and a third message type attribute to indicate that the audio link has been opened.
3. The system of clause 2, wherein the alert communicator system further comprises program instructions executable by the processor to:
   after receiving the second input, monitor the open audio link; and
   wherein, after detecting that the open audio link has been closed, generate a closed alert status update message by inserting the first message type attribute in the type attribute field.
4. The system of clause 3, wherein the alert owner system further comprises an alert monitoring database, and wherein the program instructions of the alert owner system are further executable by the processor to:
   after receiving the closed alert status update message:
   detect the first message type attribute in the type attribute field of the closed alert status update message, and
   delete the alert message associated with the closed alert status update message from the alert monitoring database.
5. The system of clause 4,
   wherein the alert owner system further comprises program instructions executable by the processor to generate, using the predetermined protocol, an alert complete message associated with the deleted alert message, and
   wherein the alert communicator system further comprises program instructions executable by the processor to, after receipt of the alert complete message, at least one of:
   remove the alert message associated with the alert complete message from a list of accepted alerts displayed on the graphical user interface; or
   move the alert message associated with the alert complete message to a list of completed alerts displayed on the graphical user interface.
6. The system of any one of clauses 2 to 5, wherein receiving the second input includes receiving a selection of one of a call icon or an accept and call icon displayed on the graphical user interface, and wherein, after selection, an opened alert status update message is generated by inserting the third message type attribute in the type attribute field.
7. The system of clause 6, wherein the alert communicator system further comprises program instructions executable by the processor to:
   after receiving the second input, hold the opened alert status update message; and
   wherein, after detecting that the open audio link has been closed, generating an opened-closed alert status update message by inserting the second message type attribute in the type attribute field.
8. The system of clause 7, wherein the alert owner system further comprises an alert monitoring database, and wherein the program instructions of the alert owner system are further executable by the processor to:
   after receiving the opened-closed alert status update message:
   detect the second message type attribute in the type attribute field of the opened-closed alert status update message, and
   delete the alert message associated with the opened-closed alert status update message from the alert monitoring database.
9. The system of clause 8,
   wherein the alert owner system further comprises program instructions executable by the processor to generate, using the predetermined protocol, an alert complete message associated with the deleted alert message, and
   wherein the alert communicator system further comprises program instructions executable by the processor to, after receipt of the alert complete message, at least one of:
   remove the alert message associated with the alert complete message from a list of accepted alerts displayed on the graphical user interface; or
   move the alert message associated with the alert complete message to a list of completed alerts displayed on the graphical user interface.
10. The system of any preceding clause, wherein receiving the first input includes receiving a selection of an accept icon displayed on the graphical user interface, and wherein, after the selection, the alert message is presented in an accepted alerts list on the graphical user interface.
11. The system of any preceding clause, wherein the alert message is associated with an alert originated in a subject's room.
12. The system of any preceding clause, wherein the audio link comprises a Voice Over Internet Protocol communication link.
13. The system of any preceding clause, wherein the predetermined communication protocol comprises wireless communications transfer protocol (WCTP).
14. An alert owner system, comprising:
   an alert monitoring database;
   a processor; and
   a non-transitory memory storing program instructions, the program instructions executable by the processor to:
      generate an alert message using a predetermined communication protocol, wherein generating the alert message includes defining at least one acceptable message type attribute acceptable in a type attribute field for alert status update messages associated with the alert message;
      after receiving a closed alert status update message including a first message type attribute:
         detect the first message type attribute in the type attribute field of the closed alert status update message, and
         delete the alert message associated with the closed alert status update message from the alert monitoring database; and
      after receiving an opened-closed alert status update message:
         detect the second message type attribute in the type attribute field of the opened-closed alert status update message, and
         delete the alert message associated with the opened-closed alert status update message from the alert monitoring database.
15. The system of clause 14, wherein the program instructions are further executable by the processor to:
   generate, using the predetermined protocol, an alert complete message associated with each deleted alert message for transmission to an alert communicator system.
16. The system of either clause 14 or clause 15, wherein the at least one acceptable message type attribute includes a first message type attribute to indicate that an audio link has been closed to a location associated with the alert message, a second message type attribute to indicate that the audio link has been opened and closed to the location associated with the alert message, and a third message type attribute to indicate that the audio link has been opened to the location associated with the alert message.
17. The system of clause 16, wherein the audio link comprises a Voice Over Internet Protocol communication link.
18. The system of any one of clauses 14 to 17, wherein the alert message is associated with an alert originated in a subject's room.
19. The system of any one of clauses 14 to 18, wherein the predetermined communication protocol comprises wireless communications transfer protocol (WCTP).
20. An alert communicator system, comprising:
   a graphical user interface;
   a processor; and
   a non-transitory memory storing program instructions, the program instructions executable by the processor to:
      receive, via the graphical user interface, an input after receipt of an alert message, wherein the input includes one of a first input or a second input, and wherein:
      after receiving the first input, present the alert message in an alert list on the graphical user interface; and
      after receiving the second input:
         open an audio link to a device at a location associated with the alert message; and
         generate at least one alert status update message using a predetermined communication protocol, wherein generating the at least one alert status update message includes inserting a message type attribute of at least one acceptable message type attribute in a type attribute field of each respective alert status update message.
21. The system of clause 20, wherein the at least one acceptable message type attribute includes a first message type attribute to indicate that the audio link has been closed, a second message type attribute to indicate that the audio link has been opened and closed, and a third message type attribute to indicate that the audio link has been opened.
22. The system of clause 21, wherein the program instructions are further executable by the processor to:
   after receiving the second input, monitor the open audio link; and
   wherein, after detecting that the open audio link has been closed, generate a closed alert status update message by inserting the first message type attribute in the type attribute field.
23. The system of clause 22, wherein the program instructions are further executable by the processor to:
   after receipt of an alert complete message, at least one of:
   remove the alert message associated with the alert complete message from a list of accepted alerts displayed on the graphical user interface; or
   move the alert message associated with the alert complete message to a list of completed alerts displayed on the graphical user interface.
24. The system of any one of clauses 21 to 23, wherein receiving the second input includes receiving a selection of one of a call icon or an accept and call icon displayed on the graphical user interface, and wherein, after selection, an opened alert status update message is generated by inserting the third message type attribute in the type attribute field.
25. The system of clause 24, wherein the program instructions are further executable by the processor to:
   after receiving the second input, hold the opened alert status update message; and
   wherein, after detecting that the open audio link has been closed, generating an opened-closed alert status update message by inserting the second message type attribute in the type attribute field.
26. The system of any one of clauses 20 to 25, wherein receiving the first input includes receiving a selection of an accept icon displayed on the graphical user interface, and wherein, after the selection, the alert message is presented in an accepted alerts list on the graphical user interface.
27. The system of any one of clauses 20 to 26, wherein the alert message is associated with an alert originated in a subject's room.
28. The system of any one of clauses 20 to 27, wherein the audio link comprises a Voice Over Internet Protocol communication link.
29. The system of any one of clauses 20 to 28, wherein the predetermined communication protocol comprises wireless communications transfer protocol (WCTP).
30. A method to generate alert status update messages in a caregiver information system, comprising:
   generating, by an alert owner system, an alert message using a predetermined communication protocol, wherein generating the alert message includes defining at least one acceptable message type attribute acceptable in a type attribute field for alert status update messages associated with the alert message; and
   receiving, via a graphical user interface of an alert communicator system, an input after receipt of the alert message, wherein the input includes one of a first input or a second input, and wherein:
      after receiving the first input, presenting the alert message in an alert list on the on the graphical user interface; and
      after receiving the second input:
         opening an audio link via the alert communicator system to a device at a location associated with the alert message; and
         generating at least one alert status update message using the communication protocol, wherein generating the at least one alert status update message includes inserting a message type attribute of the at least one acceptable message type attribute in the type attribute field of each respective alert status update message.
31. The method of clause 30, wherein the at least one acceptable message type attribute includes a first message type attribute to indicate that the audio link has been closed, a second message type attribute to indicate that the audio link has been opened and closed, and a third message type attribute to indicate that the audio link has been opened.
32. The method of clause 31, further comprising:
   after receiving the second input, monitoring, by the alert communicator system, the open audio link; and
   after detecting that the open audio link has been closed, generating, by the alert communicator system, a closed alert status update message by inserting the first message type attribute in the type attribute field.
33. The method of clause 32, further comprising:
   after receiving the closed alert status update message:
   detecting, by the alert owner system, the first message type attribute in the type attribute field of the closed alert status update message, and
   deleting, by the alert owner system, the alert message associated with the closed alert status update message from an alert monitoring database.
34. The method of clause 33, further comprising:
   generating, by the alert owner system, using the predetermined protocol, an alert complete message associated with the deleted alert message, and
   after receipt of the alert complete message, at least one of:
      removing, by the alert communicator system, the alert message associated with the alert complete message from a list of accepted alerts displayed on the graphical user interface; or
      moving, by the alert communicator system, the alert message associated with the alert complete message to a list of completed alerts displayed on the graphical user interface.
35. The method of any one of clauses 31 to 34, wherein receiving the second input includes receiving, by the alert communicator system, a selection of one of a call icon or an accept and call icon displayed on the graphical user interface, and wherein, after selection, an opened alert status update message is generated, by the alert communicator system, by inserting the third message type attribute in the type attribute field.
36. The method of clause 35, further comprising:
   after receiving the second input, holding, by the alert communicator system, the opened alert status update message; and
   after detecting that the open audio link has been closed, generating, by the alert communicator system, an opened-closed alert status update message by inserting the second message type attribute in the type attribute field.
37. The method of clause 36, wherein the alert owner system further comprises an alert monitoring database, and wherein the method further comprises:
   after receiving the opened-closed alert status update message:
   detecting, by the alert owner system, the second message type attribute in the type attribute field of the opened-closed alert status update message, and
   deleting, by the alert owner system, the alert message associated with the opened-closed alert status update message from an alert monitoring database.
38. The method of clause 37, further comprising:
   generating, by the alert owner system, using the predetermined protocol, an alert complete message associated with the deleted alert message, and
   after receipt of the alert complete message, at least one of:
      removing, by the alert communicator system, the alert message associated with the alert complete message from a list of accepted alerts displayed on the graphical user interface; or
      moving, by the alert communicator system, the alert message associated with the alert complete message to a list of completed alerts displayed on the graphical user interface.
39. The method of any one of clauses 30 to 38, wherein receiving the first input includes receiving a selection of an accept icon displayed on the graphical user interface, and wherein, after the selection, the alert message is presented in an accepted alerts list on the graphical user interface.
40. The method of any one of clauses 30 to 39, wherein the alert message is associated with an alert originated in a subject's room.
41. The method of any one of clauses 30 to 40, wherein the audio link comprises a Voice Over Internet Protocol communication link.
42. The method of any one of clauses 30 to 41, wherein the predetermined communication protocol comprises wireless communications transfer protocol (WCTP).
43. A method to delete generated alert messages, the method comprising:
   generating, by an alert owner system, an alert message using a predetermined communication protocol, wherein generating the alert message includes defining at least one acceptable message type attribute acceptable in a type attribute field for alert status update messages associated with the alert message;
   after receiving a closed alert status update message including a first message type attribute:
      detecting, by the alert owner system, the first message type attribute in the type attribute field of the closed alert status update message, and
      deleting, by the alert owner system, the alert message associated with the closed alert status update message from an alert monitoring database; and
   after receiving an opened-closed alert status update message:
      detecting, by the alert owner system, the second message type attribute in the type attribute field of the opened-closed alert status update message, and
      deleting, by the alert owner system, the alert message associated with the opened-closed alert status update message from the alert monitoring database.
44. The method of clause 43, further comprising:
   generating, by the alert owner system, using the predetermined protocol, an alert complete message associated with each deleted alert message for transmission to an alert communicator system.
45. The method of either clause 43 or clause 44, wherein the at least one acceptable message type attribute includes a first message type attribute to indicate that an audio link has been closed to a location associated with the alert message, a second message type attribute to indicate that the audio link has been opened and closed to the location associated with the alert message, and a third message type attribute to indicate that the audio link has been opened to the location associated with the alert message.
46. The method of clause 45, wherein the audio link comprises a Voice Over Internet Protocol communication link.
47. The method of any one of clauses 43 to 46, wherein the alert message is associated with an alert originated in a subject's room.
48. The method of any one of clauses 43 to 47, wherein the predetermined communication protocol comprises wireless communications transfer protocol (WCTP).
49. A method for generating alert status update messages, the method comprising:
   receiving, via a graphical user interface of an alert communicator system, an input after receipt of an alert message, wherein the input includes one of a first input or a second input, and wherein:
   after receiving the first input, presenting by the alert communicator system the alert message in an alert list on the graphical user interface; and
   after receiving the second input:
      opening, by the alert communicator system, an audio link to a device at a location associated with the alert message; and
      generating, by the alert communicator system, at least one alert status update message using a predetermined communication protocol, wherein generating the at least one alert status update message includes inserting a message type attribute of at least one acceptable message type attribute in a type attribute field of each respective alert status update message.
50. The method of clause 49, wherein the at least one acceptable message type attribute includes a first message type attribute to indicate that the audio link has been closed, a second message type attribute to indicate that the audio link has been opened and closed, and a third message type attribute to indicate that the audio link has been opened.
51. The method of clause 50, further comprising:
   after receiving the second input, monitoring, by the alert communicator system, the open audio link; and
   after detecting that the open audio link has been closed, generating by the alert communicator system a closed alert status update message by inserting the first message type attribute in the type attribute field.
52. The method of clause 51, further comprising:
   after receipt of an alert complete message, at least one of:
   removing, by the alert communicator system, the alert message associated with the alert complete message from a list of accepted alerts displayed on the graphical user interface; or
   moving, by the alert communicator system, the alert message associated with the alert complete message to a list of completed alerts displayed on the graphical user interface.
53. The method of any one of clauses 50 to 52, wherein receiving the second input includes receiving a selection of one of a call icon or an accept and call icon displayed on the graphical user interface, and wherein, after selection, an opened alert status update message is generated by inserting the third message type attribute in the type attribute field.
54. The method of clause 53, further comprising:
   after receiving the second input, holding by the alert communicator system the opened alert status update message; and
   after detecting that the open audio link has been closed, generating by the alert communicator system an opened-closed alert status update message by inserting the second message type attribute in the type attribute field.
55. The method of any one of clauses 49 to 54, wherein receiving the first input includes receiving a selection of an accept icon displayed on the graphical user interface, and wherein, after the selection, the alert message is presented in an accepted alerts list on the graphical user interface.
56. The method of any one of clauses 49 to 55, wherein the alert message is associated with an alert originated in a subject's room.
57. The method of any one of clauses 49 to 56, wherein the audio link comprises a Voice Over Internet Protocol communication link.
58. The method of any one of clauses 49 to 57, wherein the predetermined communication protocol comprises wireless communications transfer protocol (WCTP).

## Claims

1. A caregiver information system to generate alert status update messages, the system comprising:
an alert owner system, including:
a processor; and
a non-transitory memory storing program instructions, the program instructions executable by the processor to:
generate an alert message using a predetermined communication protocol, wherein generating the alert message includes defining at least one acceptable message type attribute acceptable in a type attribute field for alert status update messages associated with the alert message; and
an alert communicator system, including:
a processor; and
a non-transitory memory storing program instructions, the program instructions executable by the processor to:
receive, via a graphical user interface, an input after receipt of the alert message, wherein the input includes one of a first input or a second input, and wherein:
after receiving the first input, present the alert message in an alert list on the graphical user interface; and
after receiving the second input:
open an audio link via the alert communicator system to a device at a location associated with the alert message; and
generate at least one alert status update message using the communication protocol, wherein generating the at least one alert status update message includes inserting a message type attribute of the at least one acceptable message type attribute in the type attribute field of each respective alert status update message.

2. The system of claim 1, wherein the at least one acceptable message type attribute includes a first message type attribute to indicate that the audio link has been closed, a second message type attribute to indicate that the audio link has been opened and closed, and a third message type attribute to indicate that the audio link has been opened.

3. The system of claim 2, wherein:
the alert communicator system further comprises program instructions executable by the processor to:
after receiving the second input, monitor the open audio link; and
wherein, after detecting that the open audio link has been closed, generate a closed alert status update message by inserting the first message type attribute in the type attribute field; and
the alert owner system further comprises an alert monitoring database, and wherein the program instructions of the alert owner system are further executable by the processor to:
after receiving the closed alert status update message:
detect the first message type attribute in the type attribute field of the closed alert status update message, and
delete the alert message associated with the closed alert status update message from the alert monitoring database.

4. The system of claim 3,
wherein the alert owner system further comprises program instructions executable by the processor to generate, using the predetermined protocol, an alert complete message associated with the deleted alert message, and
wherein the alert communicator system further comprises program instructions executable by the processor to, after receipt of the alert complete message, at least one of:
remove the alert message associated with the alert complete message from a list of accepted alerts displayed on the graphical user interface; or
move the alert message associated with the alert complete message to a list of completed alerts displayed on the graphical user interface.

5. The system of any one of claims 2 to 4, wherein receiving the second input includes receiving a selection of one of a call icon or an accept and call icon displayed on the graphical user interface, and wherein, after selection, an opened alert status update message is generated by inserting the third message type attribute in the type attribute field.

6. The system of claim 5, wherein:
the alert communicator system further comprises program instructions executable by the processor to:
after receiving the second input, hold the opened alert status update message; and
wherein, after detecting that the open audio link has been closed, generating an opened-closed alert status update message by inserting the second message type attribute in the type attribute field; and
the alert owner system further comprises an alert monitoring database, and wherein the program instructions of the alert owner system are further executable by the processor to:
after receiving the opened-closed alert status update message:
detect the second message type attribute in the type attribute field of the opened-closed alert status update message, and
delete the alert message associated with the opened-closed alert status update message from the alert monitoring database.

7. The system of claim 6,
wherein the alert owner system further comprises program instructions executable by the processor to generate, using the predetermined protocol, an alert complete message associated with the deleted alert message, and
wherein the alert communicator system further comprises program instructions executable by the processor to, after receipt of the alert complete message, at least one of:
remove the alert message associated with the alert complete message from a list of accepted alerts displayed on the graphical user interface; or
move the alert message associated with the alert complete message to a list of completed alerts displayed on the graphical user interface.

8. The system as in one of claims 1-7, wherein the predetermined communication protocol comprises wireless communications transfer protocol (WCTP).

9. A method to generate alert status update messages in a caregiver information system, comprising:
generating, by an alert owner system, an alert message using a predetermined communication protocol, wherein generating the alert message includes defining at least one acceptable message type attribute acceptable in a type attribute field for alert status update messages associated with the alert message; and
receiving, via a graphical user interface of an alert communicator system, an input after receipt of the alert message, wherein the input includes one of a first input or a second input, and wherein:
after receiving the first input, presenting the alert message in an alert list on the on the graphical user interface; and
after receiving the second input:
opening an audio link via the alert communicator system to a device at a location associated with the alert message; and
generating at least one alert status update message using the communication protocol, wherein generating the at least one alert status update message includes inserting a message type attribute of the at least one acceptable message type attribute in the type attribute field of each respective alert status update message.

10. The method of claim 9, wherein the at least one acceptable message type attribute includes a first message type attribute to indicate that the audio link has been closed, a second message type attribute to indicate that the audio link has been opened and closed, and a third message type attribute to indicate that the audio link has been opened.

11. The method of claim 10, further comprising:
after receiving the second input, monitoring, by the alert communicator system, the open audio link; and
after detecting that the open audio link has been closed, generating, by the alert communicator system, a closed alert status update message by inserting the first message type attribute in the type attribute field.

12. The method of claim 11, further comprising:
after receiving the closed alert status update message:
detecting, by the alert owner system, the first message type attribute in the type attribute field of the closed alert status update message, and
deleting, by the alert owner system, the alert message associated with the closed alert status update message from an alert monitoring database; and
generating, by the alert owner system, using the predetermined protocol, an alert complete message associated with the deleted alert message, and
after receipt of the alert complete message, at least one of:
removing, by the alert communicator system, the alert message associated with the alert complete message from a list of accepted alerts displayed on the graphical user interface; or
moving, by the alert communicator system, the alert message associated with the alert complete message to a list of completed alerts displayed on the graphical user interface.

13. The method of any one of claims 10 to 12, wherein receiving the second input includes receiving, by the alert communicator system, a selection of one of a call icon or an accept and call icon displayed on the graphical user interface, and wherein, after selection, an opened alert status update message is generated, by the alert communicator system, by inserting the third message type attribute in the type attribute field.

14. The method of claim 13, further comprising:
after receiving the second input, holding, by the alert communicator system, the opened alert status update message; and
after detecting that the open audio link has been closed, generating, by the alert communicator system, an opened-closed alert status update message by inserting the second message type attribute in the type attribute field; and
after receiving the opened-closed alert status update message:
detecting, by the alert owner system, the second message type attribute in the type attribute field of the opened-closed alert status update message, and
deleting, by the alert owner system, the alert message associated with the opened-closed alert status update message from an alert monitoring database.

15. The method of claim 14, further comprising:
generating, by the alert owner system, using the predetermined protocol, an alert complete message associated with the deleted alert message, and
after receipt of the alert complete message, at least one of:
removing, by the alert communicator system, the alert message associated with the alert complete message from a list of accepted alerts displayed on the graphical user interface; or
moving, by the alert communicator system, the alert message associated with the alert complete message to a list of completed alerts displayed on the graphical user interface.
